# EUROPÄISCHE PATENTSCHRIFT

(11) **EP 4 259 228 B1**
(45) Veröffentlichungstag und Bekanntmachung des Hinweises auf die Patenterteilung: **29.07.2026**
(21) Anmeldenummer: 21836115.2
(22) Anmeldetag: 10.12.2021
(51) Int. Cl.: A61M 1/16

(54) **MEDIZINTECHNISCHE BEHANDLUNGSVORRICHTUNG UND VERFAHREN**
MEDICAL TREATMENT DEVICE AND METHOD
DISPOSITIF DE TRAITEMENT MÉDICAL ET MÉTHODE

(30) Priorität: 14.12.2020 DE 102020133387
(43) Veröffentlichungstag der Anmeldung: 18.10.2023
(73) Patentinhaber: Fresenius Medical Care Deutschland GmbH, 61352 Bad Homburg (DE)
(72) Erfinder: HAECKER, Juergen, 61267 Neu-Anspach (DE)
(74) Vertreter: Bobbert & Partner Patentanwälte PartmbB
(86) Internationale Anmeldenummer: PCT/EP2021/085232
(87) Internationale Veröffentlichungsnummer: WO 2022/128796

(56) Entgegenhaltungen:
- US-A- 5 589 070
- US-A- 5 674 404
- US-A1- 2010 243 543

## Beschreibung

Die vorliegende Erfindung betrifft ein Verfahren gemäß Anspruch 1, eine Steuervorrichtung gemäß Anspruch 11 sowie eine medizinische oder medizintechnische Behandlungsvorrichtung gemäß Anspruch 12.

Medizintechnische Behandlungsvorrichtungen, die Fluid einer externen, austauschbaren Komponente wie einem Blutfilter oder einem Dialysator mittels einer ersten Leitung zuleiten und mittels einer zweiten Leitung aus dieser Komponente ausleiten, weisen in der Praxis regelmäßig eine Kurzschlussleitung auf. Sie dient dazu, die erste Leitung mit der zweiten Leitung zu verbinden, ohne dass die genannte Komponente in den so erzeugten Kurzschlussfluidwegen eingebunden wäre, etwa zum Spülen des Systems, der beiden Leitungen, usw.

Verfahren zum Überprüfen eines Zustands einer mit einer Dialysierflüssigkeitszulaufleitung und einer Dialysatablaufleitung verbundenen Kurzschlussleitung sind z.B. aus US5589070 A bekannt.

Eine Aufgabe der vorliegenden Erfindung ist, ein weiteres Verfahren zum Überprüfen eines Zustands einer - Kurzschlussleitung, eine weitere Steuervorrichtung sowie eine weitere medizintechnische Behandlungsvorrichtung anzugeben.

Die erfindungsgemäße Aufgabe wird durch ein Verfahren mit den Merkmalen des Anspruchs 1, eine Steuervorrichtung mit den Merkmalen des Anspruchs 11 sowie durch eine medizintechnische Behandlungsvorrichtung mit den Merkmalen des Anspruchs 12 gelöst.

Erfindungsgemäß wird eine Kurzschlussleitung zum Herstellen einer Fluidverbindung zwischen wenigstens einer ersten Leitung, z. B. einer Dialysierflüssigkeitszulaufleitung, und einer zweiten Leitung, **z. B.** einer Dialysatablaufleitung einer Behandlungsvorrichtung, z. B. einer Blutbehandlungsvorrichtung, insbesondere einer Dialysiervorrichtung, vorgeschlagen.

Mittels der Dialysierflüssigkeitszulaufleitung wird im bestimmungsgemäßen Gebrauch Dialysierflüssigkeit aus dem Inneren der Behandlungsvorrichtung herausgeleitet in ein Äußeres der Behandlungsvorrichtung.

Mittels der Dialysatablaufleitung wird im bestimmungsgemäßen Gebrauch Dialysat aus dem Äußeren zurückgeführt in das Innere der Behandlungsvorrichtung.

Die erfindungsgemäße Kurzschlussleitung weist einen ersten Konnektor auf. Er dient zum Verbinden der Kurzschlussleitung mit einem ersten Konnektor der Dialysierflüssigkeitszulaufleitung, wodurch eine Fluidverbindung zwischen Kurzschlussleitung und Dialysierflüssigkeitszulaufleitung erzielt wird.

Die Kurzschlussleitung weist weiter einen zweiten Konnektor auf. Er dient zum Verbinden der Kurzschlussleitung mit einem zweiten Konnektor der Dialysatablaufleitung, wiederum unter Erzielen einer Fluidverbindung.

Außerdem weist die Kurzschlussleitung wenigstens eine Rückstromsperre, insbesondere ein Rückschlagventil, auf, welches das Durchströmen der Kurzschlussleitung mit Fluid nur oder im Wesentlichen nur in Richtung vom ersten Konnektor zum zweiten Konnektor zulässt.

Die vorstehend genannten - ersten und zweiten - Konnektoren sind in bevorzugten Ausführungsformen die einzigen Konnektoren der Kurzschlussleitung.

Erfindungsgemäß wird ein Verfahren zum Überprüfen eines Zustands einer erfindungsgemäßen Kurzschlussleitung vorgeschlagen, welche mit einer Dialysierflüssigkeitszulaufleitung und einer Dialysatablaufleitung einer bereitgestellten medizintechnischen Behandlungsvorrichtung verbunden ist. Die medizintechnische Behandlungsvorrichtung weist die Dialysierflüssigkeitszulaufleitung mit einem Konnektor auf, welcher zur Verbindung der Dialysierflüssigkeitszulaufleitung mit einem Blutfilter der medizintechnischen Behandlungsvorrichtung vorgesehen ist. Weiter weist die medizintechnische Behandlungsvorrichtung die Dialysatablaufleitung mit einem Konnektor auf, welcher zur Verbindung der Dialysatablaufleitung mit dem Blutfilter vorgesehen ist. Ergänzend weist die medizintechnische Behandlungsvorrichtung eine Fördervorrichtung zum Fördern von Fluid innerhalb der Dialysierflüssigkeitszulaufleitung und/oder innerhalb der Dialysatablaufleitung auf.

Das Verfahren soll ausgeführt werden, wenn oder nachdem die Kurzschlussleitung mittels ihres ersten Konnektors mit dem Konnektor der Dialysierflüssigkeitszulaufleitung und mittels ihres zweiten Konnektors mit dem Konnektor der Dialysatablaufleitung verbunden wurde.

Das erfindungsgemäße Verfahren umfasst ein Betätigen der Fördervorrichtung, insbesondere im Versuch oder mit dem Ziel, Fluid innerhalb oder entlang der Dialysierflüssigkeitszulaufleitung und/oder innerhalb oder entlang der Dialysatablaufleitung zu fördern, um Druck oder Unterdruck innerhalb der Dialysierflüssigkeitszulaufleitung und/oder innerhalb der Dialysatablaufleitung aufzubauen.

Das Verfahren umfasst weiter, zu einem und/oder für einen ersten Zeitpunkt, ein Messen oder anderweitiges Ermitteln des innerhalb der Dialysierflüssigkeitszulaufleitung und/oder innerhalb der Dialysatablaufleitung herrschenden Drucks. Dieser Druck wird hierin auch als Ausgangsdruck bezeichnet.

Ferner umfasst das Verfahren ein Feststellen wenigstens eines Druckwerts basierend auf dem gemessenen Druck, beispielsweise als Angabe eines Zahlenwerts mit der Dimension mbar, hPa, mmHg oder dergleichen, in Form eines Absolutwerts, eines Relativwerts, einer Differenz, usw.

Das Verfahren umfasst weiter ein Auswerten des wenigstens einen Druckwerts, etwa nach vorbestimmten Kriterien.

Ferner umfasst das Verfahren ein Abgeben oder Ausgeben eines Signals, welches den Zustand der Kurzschlussleitung angibt und ein Ergebnis des Auswertens ist.

Erfindungsgemäß wird ferner eine Steuervorrichtung vorgeschlagen, welche zum Ausführen eines Tests oder Verfahrens zum Überprüfen eines Zustands einer, vorzugsweise erfindungsgemäßen, Kurzschlussleitung programmiert ist, sofern oder wobei die Kurzschlussleitung mittels vorgenannter Konnektoren sowohl mit der Dialysierflüssigkeitszulaufleitung als auch mit der Dialysatablaufleitung einer medizintechnischen Blutbehandlungsvorrichtung verbunden ist. Der Test umfasst die Schritte des erfindungsgemäßen Verfahrens in beliebiger Ausführungsform.

Erfindungsgemäß wird weiter eine medizintechnische Behandlungsvorrichtung vorgeschlagen, welche ein Gehäuse, welches vorzugsweise ein Inneres und ein Äußeres der Behandlungsvorrichtung trennt, sowie vorzugsweise eine erfindungsgemäße Kurzschlussleitung aufweist.

Die Erfindung wird durch die Merkmale der unabhängigen Ansprüche 1 und 11 definiert. Vorteilhafte Weiterbildungen der vorliegenden Erfindung sind jeweils auch Gegenstand der Unteransprüche.

Bei allen vorstehenden oder folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Wann immer hierin räumliche Bezüge wie "oben", "unten", "oberen" oder "unteren" genannt werden, so versteht der Fachmann diese im Zweifel als eine räumlich Angabe mit Bezug auf die Ausrichtung in den hier beigefügten Figuren und/oder die Anordnung der erfindungsgemäßen Kurzschlussleitung in ihrem bestimmungsgemäßen Gebrauch.

Wenn hierin von einer Ausführungsform die Rede ist, so stellt dies stets eine erfindungsgemäße, beispielhafte Ausführungsform dar, die nicht als beschränkend zu verstehen ist.

Die Erfindung wird durch die Merkmale der unabhängigen Ansprüche 1 und 11 definiert.

Wann immer hierin eine Eignung oder ein Verfahrensschritt genannt ist umfasst die vorliegende Erfindung auch eine entsprechende Programmierung oder Konfigurierung einer hierfür oder zum Ausführen des Verfahrensschritts geeigneten und/oder programmierten Vorrichtung, oder eines Abschnitts hiervon.

Wenn hierin von einem Zeitpunkt die Rede ist, so kann dies alternativ ein Zeitraum sein.

Gemäß der beanspruchten Erfindung umfasst das Ergebnis des Auswertens eine fehlerhafte Verbindung der Kurzschlussleitung mit der Dialysierflüssigkeitszulaufleitung und/oder der Dialysatablaufleitung. In zusätzlichen Ausführungsformen ist oder umfasst das Ergebnis einen Hinweis hierauf.

Alternativ oder ergänzend ist oder umfasst das Auswerten des wenigstens einen Druckwerts, diesen mit einem, beispielsweise mit einem ersten, Referenzwert oder -bereich zu vergleichen. In jeder der hierin beschriebenen Ausführungsformen kann auf eine fehlerhafte Verbindung als Ergebnis geschlossen werden, oder wird geschlossen, wenn das Auswerten des Druckwerts, das Auswerten einer Differenz aus mehreren Druckwerten und/oder der Vergleich von Druckwerten, die für unterschiedliche Leitungen festgestellt wurden, ergibt, dass ein hierfür festgelegter bzw. vorbestimmter Referenzwert überschritten wird oder der Druckwert außerhalb eines hierfür festgelegten oder vorbestimmten Referenzbereichs liegt.

Referenzwerte und/oder Referenzbereiche können in bestimmten Ausführungsformen derart ausgestaltet sein, dass auf eine fehlerhafte Verbindung als Ergebnis geschlossen werden kann, wenn das Auswerten wie oben ausgeführt ergibt, dass ein hierfür festgelegter bzw. vorbestimmter Referenzwert unterschritten wird oder der Druckwert innerhalb eines hierfür festgelegten bzw. vorbestimmten Referenzbereichs liegt.

In manchen Ausführungsformen kann auf eine fehlerfreie Verbindung als Ergebnis geschlossen werden, oder wird geschlossen, wenn das Auswertens des Druckwerts ergibt, dass dieser einen Referenzwert, z. B. den ersten Referenzwert, nicht überschreitet oder innerhalb, beispielsweise des ersten, Referenzbereichs liegt, oder umgekehrt. Referenzwerte oder -bereiche können Grenzwerte sein oder umfassen oder von solchen begrenzt sein.

Gemäß der beanspruchten Erfindung ist das Ergebnis eine fehlerhafte Funktion der Rückstromsperre der Kurzschlussleitung. In zusätzlichen Ausführungsformen ist oder umfasst das Ergebnis einen Hinweis hierauf.

Alternativ oder ergänzend ist oder umfasst das Ergebnis, dass eine fehlerhafte Verbindung vorliegt. Dies kann durch eine Verbindung des Konnektors der Dialysierflüssigkeitszulaufleitung mit dem zweiten Konnektor der Kurzschlussleitung, welcher mit der Dialysatablaufleitung verbunden sein sollte, und eine Verbindung des Konnektors der Dialysatablaufleitung mit dem ersten Konnektor der Kurzschlussleitung, welcher mit der Dialysierflüssigkeitszulaufleitung verbunden sein sollte, begründet sein, Folge hiervon sein oder diese Konfiguration voraussetzen.

In einigen Ausführungsformen des erfindungsgemäßen Verfahrens wird oder wurde beim Feststellen des Druckwerts sowohl der zum ersten Zeitpunkt in der Dialysierflüssigkeitszulaufleitung herrschende Druck als auch der zum ersten Zeitpunkt in der Dialysatablaufleitung herrschende Druck gemessen. In diesen, oder auch anderen, Ausführungsformen wird vorzugsweise zwischen dem in der Dialysierflüssigkeitszulaufleitung gemessenen Druck oder festgestellten Druckwert und dem in der Dialysatablaufleitung gemessenen Druck oder festgestellten Druckwert, oder umgekehrt, eine Druckdifferenz ermittelt.

Das Auswerten des wenigstens einen Druckwerts ist oder umfasst in diesen Ausführungsformen ein Vergleichen dieser Druckdifferenz beispielsweise mit einem zweiten Referenzwert oder -bereich hierfür.

In bestimmten Ausführungsformen kann als Ergebnis auf eine fehlerhafte Verbindung geschlossen werden, wenn das Auswerten des Druckwerts ergibt, dass der Druckwert den zweiten Referenzwert überschreitet oder außerhalb des zweiten Referenzbereichs liegt.

In manchen Ausführungsformen umfasst das erfindungsgemäße Verfahren als weiteren Schritt ein Messen eines in der Dialysierflüssigkeitszulaufleitung und/oder in der Dialysatablaufleitung herrschenden Drucks zu einem zweiten Zeitpunkt, welcher zeitlich nach dem ersten Zeitpunkt liegt. Ergänzend umfasst es ein Feststellen wenigstens eines zweiten, hierauf basierenden Druckwerts.

In diesen Ausführungsformen ist oder umfasst das Auswerten des Druckwerts ein Vergleichen der Druckdifferenz zwischen den in der Dialysierflüssigkeitszulaufleitung zum ersten und zweiten Zeitpunkt gemessenen Drücken oder hierauf basierend festgestellten Druckwerten und/oder ein Vergleichen der Druckdifferenz zwischen den in der Dialysatablaufleitung zum ersten und zweiten Zeitpunkt gemessenen Drücken oder hierauf basierend festgestellten Druckwerten, also vorzugsweise unter Berücksichtigung der Ausgangsdrücke, mit jeweils einem, beispielsweise einem dritten bzw. vierten, Referenzwert oder -bereich hierfür.

In bestimmten Ausführungsformen kann als Ergebnis auf eine fehlerhafte Verbindung geschlossen werden, wenn das Auswerten der Druckwerte ist oder umfasst, dass wenigstens einer von ihnen den dritten bzw. vierten Referenzwert überschreitet oder außerhalb des dritten bzw. vierten Referenzbereichs liegt.

In einigen Ausführungsformen erfolgt das Betätigen der Fördereinrichtung mit dem Ziel, sowohl in der Dialysierflüssigkeitszulaufleitung als auch in der Dialysatablaufleitung Unterdruck zu erzeugen. Ergänzend umfasst das erfindungsgemäße Verfahren den weiteren Schritt eines Entspannens des erzeugten Unterdrucks in der Dialysatablaufleitung.

In diesen Ausführungsformen umfasst das Messen eines in der Dialysierflüssigkeitszulaufleitung und/oder in der Dialysatablaufleitung herrschenden Drucks ein Messen zu einem oder zu mehreren Zeitpunkten nach dem Entspannen.

In manchen Ausführungsformen erfolgt das Betätigen der wenigstens einen Fördervorrichtung und/oder der Aufbau von Überdruck oder Unterdruck bei einem geöffneten ersten Ventil, welches stromauf des Konnektors der Dialysierflüssigkeitszulaufleitung, aber stromab des ersten Drucksensors, angeordnet ist. Alternativ oder ergänzend, erfolgt das Betätigen oder der Aufbau bei einem geöffneten zweiten Ventil, welches stromab des zweiten Konnektors der Dialysatablaufleitung, aber stromauf des zweiten Drucksensors, angeordnet ist.

In einigen Ausführungsformen erfolgt das Entspannen des in der Dialysatablaufleitung herrschenden Unterdrucks vorzugsweise durch ein Verbinden der Dialysatablaufleitung mit einer Frischwasserquelle oder einem Abfluss oder umfasst dieses Verbinden.

Noch bevorzugter erfolgt das Entspannen des Unterdrucks mittels Öffnens eines Ventils zu einer Frischwasserleitung oder eines Ventils zu einer Drain- oder Abflussleitung oder umfasst dieses Öffnen.

In manchen Ausführungsformen ist das ab- oder ausgegebene Signal einem fehlerhaften Zustand der Kurzschlussleitung zugeordnet oder codiert hierfür.

Dies kann insbesondere der Fall sein, wenn das Auswerten des Druckwerts dessen Überschreiten eines Referenzwertes oder Verlassen eines Referenzbereichs ergibt oder umfasst. Alternativ oder ergänzend, wenn das Feststellen des Druckwerts basierend auf gemessenen Drücken, welche nach der Entspannung innerhalb der Dialysierflüssigkeitszulaufleitung gemessen wurden, eine Druckerhöhung ergibt.

In einigen Ausführungsformen umfasst das erfindungsgemäße Verfahren weiter ein fluidisches Absperren der Dialysatablaufleitung, oder eines Abschnitts hiervon, nach dem Entspannen des Unterdrucks in der Dialysatablaufleitung derart, dass keine Verbindung der Dialysatablaufleitung oder des Abschnitts zur Frischwasserquelle oder dem Abfluss mehr besteht.

In diesen Ausführungsformen werden zum Feststellen des wenigstens einen Druckwerts zumindest zwei Drücke in der Dialysatablaufleitung zu zwei unterschiedlichen Zeitpunkten nach ihrem Sperren gemessen.

Ergänzend wird in diesen Ausführungsformen ein Signal ab- oder ausgegeben, welches einem fehlerhaften Zustand der Kurzschlussleitung zugeordnet ist, insbesondere wenn das Auswerten des wenigstens einen Druckwerts als Ergebnis eine Druckänderung innerhalb der Dialysatablaufleitung feststellt.

In manchen Ausführungsformen der erfindungsgemäßen medizintechnischen Behandlungsvorrichtung kann die Kurzschlussleitung, zumindest abschnittsweise, im Inneren oder im Gehäuse der Behandlungsvorrichtung aufgenommen sein. Hierbei sind der erste Konnektor und der zweite Konnektor der Kurzschlussleitung vorzugsweise von einem Äußeren der Behandlungsvorrichtung für den Nutzer für eine Verbindung wie hierin beschrieben zugänglich.

In einigen Ausführungsformen der medizintechnischen Behandlungsvorrichtung weist diese eine erfindungsgemäße Steuervorrichtung auf.

Alternativ oder ergänzend weist die medizintechnische Behandlungsvorrichtung eine Dialysierflüssigkeitszulaufleitung auf mit einem zu ihrer Verbindung mit einem Blutfilter vorgesehenen Konnektor, eine Dialysatablaufleitung mit einem zu ihrer Verbindung mit einem Blutfilter vorgesehenen Konnektor und eine Fördervorrichtung zum Fördern von Fluid innerhalb der Dialysierflüssigkeitszulaufleitung und/oder innerhalb der Dialysatablaufleitung.

Als Zustand der erfindungsgemäßen Kurzschlussleitung kann hierin beispielsweise ihr Vorhandensein, ihre Unversehrtheit, ihre korrekte Anordnung an der medizintechnischen Behandlungsvorrichtung, ihre korrekte Verbindung mit der Dialysierflüssigkeitszulaufleitung und/oder mit der Dialysatablaufleitung und/oder eine Funktionsfähigkeit der Kurzschlussleitung, insbesondere eine Funktionsfähigkeit der in ihr angeordneten Rückstromsperre, verstanden werden.

In manchen Ausführungsformen ist oder umfasst das Auswerten des wenigstens einen Druckwerts ein Ermitteln einer Veränderung des Drucks, beispielsweise durch Vergleich mit Grenzwerten, Schwellenwerten, Wertebereichen und/oder ist oder umfasst ein Ermitteln der Dauer, bis eine Veränderung eintritt, usw.

Ferner umfasst in bestimmten Ausführungsformen das Verfahren das Auswerten der ermittelten Veränderung anhand von vorgegebenen Kriterien oder Größen wie z. B. Grenzwerten, Schwellenwerten, Wertebereichen und/oder der Dauer, bis die, oder eine, vorbestimmte Druckveränderung eingetreten ist, usw.

Die vorgegebenen Kriterien und Größen können beispielsweise in einer Speichervorrichtung, etwa der medizintechnischen Behandlungsvorrichtung, hinterlegt sein und von dort ausgelesen werden.

In einigen Ausführungsformen kann das erfindungsgemäße Verfahren als weiteren Schritt eine optionale Ausgabe des Signals oder eines entsprechenden Signals an den Benutzer der medizintechnischen Behandlungsvorrichtung umfassen. Das Signal an den Benutzer, etwa optisch, akustisch oder dergleichen, kann dem Benutzer mitteilen, dass das Auswerten, z. B. die ermittelte Veränderung, vorbestimmten Bedingungen genügt oder eben nicht genügt.

In einigen Ausführungsformen erfolgt das Messen von Drücken, welches zum ersten Zeitpunkt und/oder zum zweiten Zeitpunkt stattfindet, und/oder mit welchem der innerhalb der Dialysierflüssigkeitszulaufleitung und/oder innerhalb der Dialysatablaufleitung herrschende Ausgangsdruck bzw. Druck gemessen wird, mittels - oder unter Einsatz - eines stromauf des ersten Konnektors in oder an der Dialysierflüssigkeitszulaufleitung angeordneten ersten Drucksensors und/oder mittels eines stromab des zweiten Konnektors in oder an der Dialysatablaufleitung angeordneten zweiten Drucksensors.

In manchen Ausführungsformen ist die wenigstens eine Fördervorrichtung eine Ultrafiltrationspumpe und/oder eine zweite Flusspumpe, welche stromab der Dialysatablaufleitung angeordnet **ist.**

In einigen Ausführungsformen umfasst das Betätigen der Fördervorrichtung ein Aufbauen eines Unterdrucks oder Drucks sowohl stromauf des Konnektors der Dialysierflüssigkeitszulaufleitung als auch stromab des Konnektors der Dialysatablaufleitung.

In einigen Ausführungsformen kann das Betätigen der wenigstens einen Fördervorrichtung zum ersten Zeitpunkt derart erfolgen, dass vorzugsweise am ersten Drucksensor und am zweiten Drucksensor im Wesentlichen gleiche Druckwerte anliegen.

In manchen Ausführungsformen der erfindungsgemäßen medizintechnischen Behandlungsvorrichtung kann zwischen dem ersten Zeitpunkt und dem zweiten Zeitpunkt stromab des zweiten Konnektors und/oder zur Dialysatablaufleitung eine Verbindung zur Atmosphäre hergestellt werden.

In bestimmten Ausführungsformen der vorliegenden Erfindung ist das geförderte Fluid eine Dialysierflüssigkeit, in manchen erfindungsgemäßen Ausführungsformen eine von einer Behandlungsvorrichtung online her- und/oder bereitgestellte Dialysierflüssigkeit.

Die erfindungsgemäße Behandlungsvorrichtung ist in bestimmten Ausführungsformen als extrakorporale Behandlungsvorrichtung, insbesondere als extrakorporale Blutbehandlungsvorrichtung, etwa als Dialysiervorrichtung, insbesondere als Hämodialysevorrichtung, Hämofiltrationsvorrichtung, Hämodiafiltrationsvorrichtung, usw. ausgestaltet.

Die vorliegende Erfindung betrifft ferner ein digitales, insbesondere nicht-flüchtiges, Speichermedium, insbesondere in Form eines maschinenlesbaren Trägers, insbesondere in Form einer Diskette, Speicherkarte, CD, DVD, EPROM, FRAM (Ferroelectric RAM) oder SSD (Solid-State-Drive), insbesondere mit elektronisch oder optisch auslesbaren Steuersignalen. Dieses kann derart mit einem programmierbaren Computersystem zusammenwirken, dass eine Steuervorrichtung zu einer erfindungsgemäßen Steuervorrichtung konfiguriert wird, mittels welcher die, insbesondere maschinellen, Schritte des erfindungsgemäßen Verfahrens veranlasst werden können. Alternativ kann das Speichermedium konfiguriert sein, mit einer herkömmlichen medizintechnischen Behandlungsvorrichtung derart zusammenzuwirken, dass diese zu einer erfindungsgemäßen medizintechnischen Behandlungsvorrichtung umprogrammiert werden kann.

Die vorliegende Erfindung betrifft ferner ein Computerprogramm-Produkt, welches einen volatilen, flüchtigen oder auf einem maschinenlesbaren Träger gespeicherten Programmcode oder eine Signalwelle aufweist, welche geeignet sind, eine Steuervorrichtung zu einer erfindungsgemäßen Steuervorrichtung zu konfigurieren, mittels welcher die, insbesondere maschinellen, Schritte des erfindungsgemäßen Verfahrens veranlasst werden können, wenn das Computerprogramm-Produkt auf einem Rechner abläuft. Alternativ kann das Computerprogramm-Produkt konfiguriert sein, mit einer herkömmlichen medizintechnischen Behandlungsvorrichtung derart zusammenzuwirken, dass diese zu einer erfindungsgemäßen medizintechnischen

Behandlungsvorrichtung umprogrammiert werden kann.

Unter einem Computerprogramm-Produkt kann erfindungsgemäß beispielsweise ein auf einem Träger gespeichertes Computerprogramm, ein Embedded System als umfassendes System mit einem Computerprogramm (z. B. elektronisches Gerät mit einem Computerprogramm), ein Netzwerk von computerimplementierten Computerprogrammen (z. B. Client/Server-system, Cloud Computing System, etc.) oder ein Computer, auf dem ein Computerprogramm geladen ist, abläuft, gespeichert ist, ausgeführt oder entwickelt wird, verstanden werden.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen, sowie jeder andere hierin genannte Speicher oder jedes andere hierin genannte Speichermedium sein.

Die vorliegende Erfindung betrifft außerdem ein Computerprogramm, welches einen Programmcode umfasst, mittels welchem eine herkömmliche Steuervorrichtung zu einer erfindungsgemäßen Steuervorrichtung konfiguriert werden kann, mittels welcher die, insbesondere maschinellen, Schritte des erfindungsgemäßen Verfahrens veranlasst werden können. Alternativ ist das Computerprogramm geeignet, mit einer herkömmlichen medizintechnischen Behandlungsvorrichtung derart zusammenzuwirken, dass diese zu einer erfindungsgemäßen medizintechnischen Behandlungsvorrichtung umprogrammiert werden kann.

Manche oder alle erfindungsgemäßen Ausführungsformen können einen, mehrere oder alle der vorstehend und/oder im Folgenden genannten Vorteile aufweisen.

Ein Vorteil der vorliegenden Erfindung kann darin bestehen, das Risiko einer Kreuzkontamination von einer Seite, z. B. der Dialysatseite, zur anderen Seite, z. B. der Hydraulikseite, der Behandlungsvorrichtung zu verringern oder ganz zu eliminieren. Da mittels der Rückstromsperre ein Vertauschen der Konnektoren der Kurzschlussleitung mittels Überwachen des Druckverlaufs zu erkennen ist, kann im Falle von vertauschten Konnektoren auf die Notwendigkeit einer Desinfektion vor der nächsten Behandlung hingewiesen werden.

Mittels der vorliegenden Erfindung kann weiter vorteilhaft die Funktionsfähigkeit der Kurzschlussleitung geprüft und ein entsprechender Hinweis an den Benutzer kommuniziert werden.

Alle mit dem erfindungsgemäßen Verfahren erzielbaren Vorteile lassen sich in bestimmten erfindungsgemäßen Ausführungsformen ungeschmälert auch mit den erfindungsgemäßen Vorrichtungen erzielen, und umgekehrt.

Im Folgenden wird die vorliegende Erfindung unter Bezugnahme auf die beigefügten Figuren beschrieben. In der Zeichnung bezeichnen identische Bezugszeichen identische oder ähnliche Elemente. Es gilt:
- **Fig. 1**: zeigt schematisch vereinfacht einen Fluidleitungsaufbau einer erfindungsgemäßen medizintechnischen Behandlungsvorrichtung;
- **Fig. 2**: zeigt schematisch vereinfacht einen Ausschnitt des Fluidleitungsaufbaus der Fig. 1 mit einer erfindungsgemäßen Kurzschlussleitung; und
- **Fig. 3**: zeigt schematisch vereinfacht den Ablauf des erfindungsgemäßen Verfahrens in einer Ausführungsform.

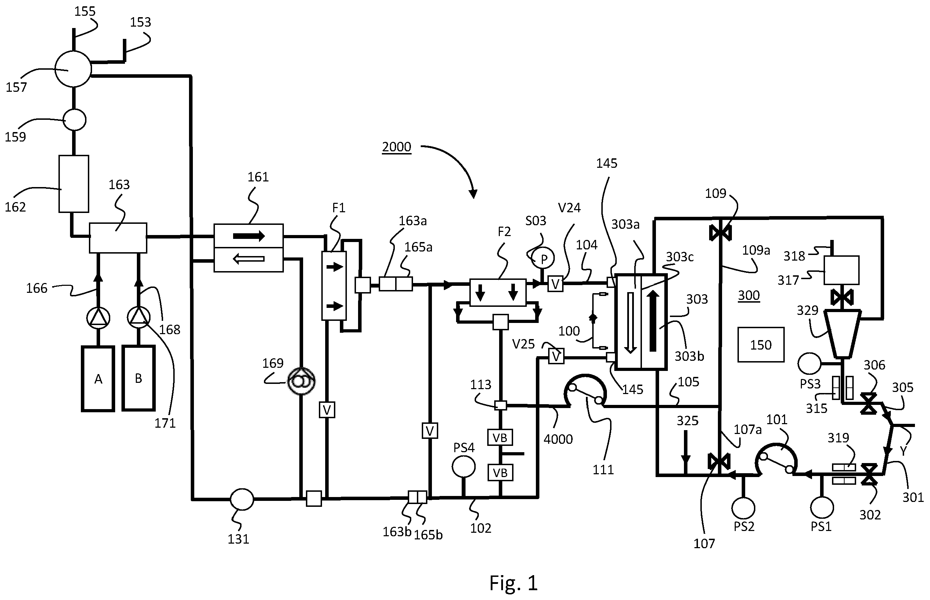

**Fig. 1** zeigt ein Verfahrensschaubild einer erfindungsgemäßen medizinischen oder medizintechnischen Behandlungsvorrichtung 2000, hier einer Blutbehandlungsvorrichtung, verbunden mit einem extrakorporalen Blutkreislauf 300, welcher zu einer Behandlung mittels Double-Needle-Zugangs, oder unter Verwendung z. B. eines zusätzlichen Y-Verbinders (Bezugszeichen Y) wie in Fig. 1 gezeigt mittels Single-Needle-Zugangs mit dem Gefäßsystem des nicht dargestellten Patienten verbunden werden kann. Der Blutkreislauf 300 kann optional in Abschnitten hiervon in oder auf einer Blutkassette vorliegen.

Pumpen, Aktoren und/oder Ventile im Bereich des Blutkreislaufs 300 sind mit der erfindungsgemäßen Behandlungsvorrichtung 2000 bzw. mit einer von dieser z. B. umfassten Steuervorrichtung 150 verbunden.

Der Blutkreislauf 300 weist eine arterielle Patientenschlauchklemme 302 und eine arterielle Konnektionsnadel eines arteriellen Abschnitts oder einer arteriellen Patientenleitung, Blutentnahmeleitung oder ersten Leitung 301 auf (oder ist hiermit verbunden). Der Blutkreislauf 300 weist ferner eine venöse Patientenschlauchklemme 306 und eine venöse Konnektionsnadel eines venösen Abschnitts, einer venösen Patientenleitung, Blutrückgabeleitung oder zweiten Leitung 305 auf (oder ist hiermit verbunden).

Eine Blutpumpe 101 ist in oder an der ersten Leitung 301 vorgesehen, eine Substituatpumpe 111 ist mit einer Dialysierflüssigkeitszulaufleitung 104 zum Fördern von frischer Dialysierflüssigkeit, welche in einer weiteren Filterstufe (F2) gefiltert ist (Substituat), verbunden. Eine Substituatleitung 105 kann fluidisch mit der Zulaufleitung 104 verbunden sein. Mittels der Substituatpumpe 111 kann Substituat per Prädilution, über ein Prädilutionsventil 107, oder per Postdilution, über ein Postdilutionsventil 109, über zugehörige Leitungen 107a bzw. 109a in Leitungsabschnitte, beispielsweise in den arteriellen Leitungsabschnitt 301 bzw. in den venösen Leitungsabschnitt 305 (hier zwischen einer Blutkammer 303b eines Blutfilters 303 und einer venösen Luftabscheidekammer oder venösen Blutkammer 329) des Blutkreislaufs 300 eingebracht werden.

Der Blutfilter 303 weist die mit dem arteriellen Leitungsabschnitt 301 und mit dem venösen Leitungsabschnitt 305 verbundene Blutkammer 303b auf. Eine Dialysierflüssigkeitskammer 303a des Blutfilters 303 ist mit der zur Dialysierflüssigkeitskammer 303a führenden Dialysierflüssigkeitszulaufleitung 104 und einer von der Dialysierflüssigkeitskammer 303a wegführenden Dialysatablaufleitung 102, welche Dialysat, also verbrauchte Dialysierflüssigkeit, leitet, verbunden. Hierzu dienen geeignete Konnektoren 145, 147 an der Dialysierflüssigkeitszulaufleitung 104 und an der Dialysatablaufleitung 102.

Dialysierflüssigkeitskammer 303a und Blutkammer 303b sind durch eine zumeist semi-permeable Membran 303c voneinander getrennt. Sie stellt die Trennscheide zwischen der Blutseite mit dem extrakorporalen Blutkreislauf 300 und der Maschinenseite mit dem Dialysierflüssigkeits- bzw. Dialysatkreislauf dar, die in Fig. 1 links der Membran 303c gezeigt ist.

Die Anordnung der Fig. 1 umfasst ferner ein Ventil V24, welches in der Dialysierflüssigkeitszulaufleitung 104 stromauf des ersten Konnektors 145, aber stromab eines ersten Drucksensors S03 angeordnet ist. Sie umfasst weiter ein Ventil V25, welches in der Dialysatablaufleitung 102, stromab des zweiten Konnektors 147, aber stromauf eines zweiten Drucksensors PS4, angeordnet ist.

Die Anordnung der Fig. 1 umfasst einen optionalen Detektor 315 zum Detektieren von Luft und/oder Blut. Die Anordnung der Fig. 1 umfasst ferner einen oder zwei Drucksensoren PS1 (stromauf der Blutpumpe 101) und PS2 (stromab der Blutpumpe 101, er misst den Druck stromauf des Blutfilters 303 ("prä-Hämofilter")) an den in Fig. 1 gezeigten Stellen. Weitere Drucksensoren können vorgesehen sein, z. B. der Drucksensor PS3 stromab der venösen Blutkammer 329.

Eine optionale Single-Needle-Kammer 317 kommt in Fig. 1 als Puffer- und/oder Ausgleichsbehälter bei einem Single-Needle-Verfahren zum Einsatz, bei welchem der Patient mittels nur einer der zwei Blutleitungen 301, 305 mit dem extrakorporalen Blutkreislauf 300 verbunden ist.

Die Anordnung der Fig. 1 umfasst außerdem einen optionalen Detektor 319 zum Detektieren von Luftblasen und/oder Blut.

Eine Zugabestelle 325 für Heparin kann optional vorgesehen sein.

Links in Fig. 1 ist eine Anmischvorrichtung 163 gezeigt, welche aus den Behältern A (für A-Konzentrat über die Konzentratversorgung 166) und B (für B-Konzentrat über die Konzentratversorgung 168) eine vorbestimmte Mischung für die jeweilige Lösung zur Verwendung durch die Behandlungsvorrichtung 2000 bereitstellt. Die Lösung enthält, in z. B. der Heizvorrichtung 162 angewärmtes Wasser (online, z. B. als Umkehrosmosewasser oder aus Beuteln) aus der Wasserquelle 155.

Eine Pumpe 171, welche als Konzentratpumpe oder Natriumpumpe bezeichnet werden kann, ist mit der Anmischvorrichtung 163 und einer Quelle mit Natrium, etwa dem Behälter A, fluidisch verbunden und/oder fördert hieraus. Eine optionale Pumpe 173, welche dem Behälter B, etwa für Bicarbonat, zugeordnet ist, ist zu erkennen.

Der hierin genannte, optionale Kompressor ist in Fig. 1 nicht gezeigt.

Weiter ist in Fig. 1 ein Abfluss 153 für das Effluent zu erkennen. Ein optionaler Wärmetauscher 157 und eine erste Flusspumpe 159, die zur Entgasung geeignet ist, ergänzen die gezeigte Anordnung.

Der Drucksensor PS4 stromab des Blutfilters 303 auf der Wasserseite, jedoch vorzugsweise stromauf der Ultrafiltrationspumpe 131 in der Dialysatablaufleitung 102 kann zum Messen des Drucks in der Dialysatablaufleitung 102, beispielsweise zum Messen des Filtratdrucks oder Membrandrucks des Blutfilters 303, vorgesehen sein.

Blut, das den Blutfilter 303 verlässt, durchströmt eine optionale venöse Blutkammer 329, welche eine Entlüftungseinrichtung 318 aufweisen und mit dem Drucksensor PS3 in Fluidverbindung stehen kann.

Die in Fig. 1 gezeigte exemplarische Anordnung weist die Steuer- oder Regelvorrichtung 150 auf. Sie kann mit jeder der hierin genannten Komponenten - jedenfalls oder insbesondere mit der Blutpumpe 101 - in kabelgebundener oder kabelloser Signalverbindung zur Steuerung oder Regelung der Behandlungsvorrichtung 2000 stehen.

Mittels der Vorrichtung zur Online-Mischung der Dialysierflüssigkeit ist eine Variation deren Natriumgehalts, gesteuert durch die Steuervorrichtung 150, in bestimmten Grenzen möglich. Hierzu können insbesondere die mittels Leitfähigkeitssensoren 163a, 163b ermittelten Messwerte einbezogen werden. Sollte sich dabei eine Anpassung des Natriumgehalts der Dialysierflüssigkeit (Natriumkonzentration) oder des Substituats als erforderlich oder gewünscht ergeben, so kann dies durch Anpassen der Förderrate der Natriumpumpe 171 erfolgen.

Darüber hinaus umfasst die Behandlungsvorrichtung 2000 Mittel zur Förderung von frischer Dialysierflüssigkeit sowie von Dialysat. Ein erstes Ventil V24 kann zwischen der ersten Flusspumpe 159 und dem Blutfilter 303 vorgesehen sein, welches eingangsseitig den Zulauf zum Blutfilter 303 öffnet bzw. schließt. Eine zweite, optionale Flusspumpe 169 ist z. B. stromabwärts des Blutfilters 303 vorgesehen, welche Dialysat zum Abfluss 153 fördert. Ein zweites Ventil kann zwischen dem Blutfilter 303 und der zweiten Flusspumpe 169 vorgesehen sein, welches ausgangsseitig den Ablauf öffnet bzw. schließt.

Weiterhin umfasst die Behandlungsvorrichtung 2000 optional eine Vorrichtung 161 zur Bilanzierung des in den Blutfilter 303 ein- und ausströmenden Flusses auf der Maschinenseite. Die Vorrichtung 161 zur Bilanzierung ist vorzugsweise in einem Leitungsbereich zwischen der ersten Flusspumpe 159 und der zweiten Flusspumpe 169 angeordnet.

Die Behandlungsvorrichtung 2000 umfasst ferner Mittel zum exakten Entfernen eines durch den Anwender und/oder durch die Steuervorrichtung 150 vorgegebenen Flüssigkeitsvolumens aus dem bilanzierten Kreislauf wie die Ultrafiltrationspumpe 131.

Sensoren wie die optionalen Leitfähigkeitssensoren 163a, 163b dienen der Bestimmung der, in manchen Ausführungsformen temperaturkompensierten, Leitfähigkeit sowie des Flüssigkeitsstroms stromauf und stromab des Blutfilters 303.

Temperatursensoren 165a, 165b können einzeln oder zu mehreren vorgesehen sein. Von ihnen gelieferte Temperaturwerte können erfindungsgemäß zum Ermitteln einer temperaturkompensierten Leitfähigkeit genutzt werden.

Weitere Flusspumpen, ergänzend oder alternativ zu z. B. jener mit dem Bezugszeichen 169, können ebenfalls vorgesehen sein.

Eine Reihe von optionalen Ventilen ist in Fig. 1 jeweils mit V bezeichnet. Bypassventile sind mit VB bezeichnet.

Basierend auf den Messwerten der vorgenannten, optionalen Sensoren ermittelt in einigen Ausführungsformen die Steuervorrichtung 150 die Elektrolyt- und/oder Flüssigkeitsbilanz.

Filter F1 und F2 können in Serie geschaltet vorgesehen sein.

Der Filter F1 dient hier exemplarisch dazu, mittels der Anmischvorrichtung 163 selbst unter Nutzung von nicht-reinem Wasser ausreichend reine Dialysierflüssigkeit zu erzeugen, welche anschließend, z. B. im Gegenstromprinzip, durch den Blutfilter 303 strömt.

Der Filter F2 dient hier exemplarisch dazu, aus der ausreichend reinen Dialysierflüssigkeit, welche den ersten Filter F1 verlässt, durch Filtern von z. B. pyrogenen Stoffen steriles oder ausreichend gefiltertes Substituat zu generieren, welches bedenkenlos dem extrakorporal strömenden Blut des Patienten und damit letztlich dem Körper des Patienten zugeführt werden kann.

Die Behandlungsvorrichtung 2000 ist in Fig. 1 zwar optional als Vorrichtung zur Hämo(dia)filtration gezeigt. Hämodialysevorrichtungen fallen jedoch ebenfalls unter die vorliegende Erfindung, obgleich nicht eigens mittels Figur dargestellt.

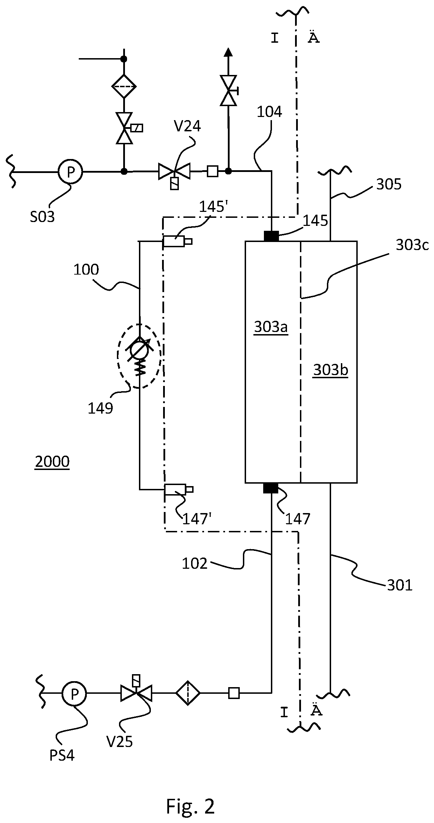

Zu erkennen ist eine mögliche Position der erfindungsgemäßen Kurzschlussleitung 100 innerhalb der Behandlungsvorrichtung 2000. Sie wird zu Fig. 2 näher beschrieben.

Die vorliegende Erfindung ist nicht auf die vorstehend beschriebene Ausführungsform beschränkt, diese dient lediglich der Veranschaulichung.

Die in Fig. 1 gezeigten Pfeilspitzen geben in Fig. 1 allgemein jeweils die Strömungsrichtung an.

**Fig.** 2 zeigt schematisch vereinfacht einen Ausschnitt des Fluidleitungsaufbaus der Fig. 1 mit einer erfindungsgemäßen Kurzschlussleitung 100. Es wird deshalb auf die Beschreibung zur Fig. 1 Bezug genommen.

Die Kurzschlussleitung 100 ist dazu ausgestaltet bzw. vorgesehen, nach Beenden einer Behandlungssitzung mit ihrem ersten Ende mittels dessen Konnektors 145' mit dem ersten Konnektor 145 der Dialysierflüssigkeitszulaufleitung 104 der Behandlungsvorrichtung 2000 sowie mit ihrem zweiten Ende mittels dessen Konnektors 147' mit dem zweiten Konnektor der Dialysatablaufleitung 102 verbunden zu werden mit dem Ziel, mit den jeweiligen Leitungen 102, 104 eine Fluidverbindung herzustellen bzw. diese kurzzuschließen.

Mittels der Dialysierflüssigkeitszulaufleitung 104 der Behandlungsvorrichtung 2000 wird bestimmungsgemäß Dialysierflüssigkeit aus dem Inneren I der Behandlungsvorrichtung 2000 und in ein Äußeres Ä der Behandlungsvorrichtung 2000 herausgeleitet, beziehungsweise Dialysierflüssigkeit von der Behandlungsvorrichtung 2000 dem Blutfilter 303 zugeführt.

Mittels der Dialysatablaufleitung 102 der Behandlungsvorrichtung 2000 wird bestimmungsgemäß Dialysat aus dem Äußeren Ä in das Innere I der Behandlungsvorrichtung 2000 zurückgeführt, beziehungsweise Dialysat aus dem Blutfilter 303 wieder der Behandlungsvorrichtung 2000 zugeführt.

Inneres I und Äußeres Ä sind in der Fig. 2 mittels einer Strichpunktlinie getrennt angedeutet.

Die Kurzschlussleitung 100 weist ferner wenigstens eine Rückstromsperre 149 auf, hier rein exemplarisch ein Rückschlagventil, welche das Durchströmen der Kurzschlussleitung 100 nur, oder im Wesentlichen nur, in Richtung von deren erstem Konnektor 145' zu deren zweitem Konnektor 147' zulässt oder ein Durchströmen in der entgegengesetzten Richtung behindert oder verhindert. Damit kann verhindert werden, dass es nach der Behandlung und nach dem bestimmungsgemäßen Verbinden sowohl der Dialysierflüssigkeitszulaufleitung 104 mit der Kurzschlussleitung 100 als auch der Dialysatablaufleitung 102 mit der Kurzschlussleitung 100 entlang der Kurzschlussleitung 100 und in dieser einen Fluss von Fluid von der Dialysatablaufleitung 102 zur Dialysierflüssigkeitszulaufleitung 104 geben kann. Verhindert wird im bestimmungsgemäßen Gebrauch somit eine Strömung ausgehend von der Dialysatablaufleitung 102 in Richtung zur Dialysierflüssigkeitszulaufleitung 104, beziehungsweise eine fluidische Verbindung ausgehend vom Konnektor der Dialysatablaufleitung 147 in Richtung zum Konnektor der Dialysierflüssigkeitszulaufleitung 145, nicht aber in umgekehrter Richtung.

Bei einem Vertauschen der Konnektoren 145' und 147' beim Verbinden, das heißt bei einem versehentlichen Verbinden des ersten Konnektors 145' der Kurzschlussleitung 100 mit dem Konnektor der Dialysatablaufleitung 147 und folglich einem Verbinden des zweiten Konnektors 147' der Kurzschlussleitung 100 mit dem Konnektor der Dialysierflüssigkeitszulaufleitung 145, was einem Zustand der Kurzschlussleitung 100 entspricht, würde hingegen eine Fluidströmung innerhalb der Kurzschlussleitung 100 von dem zweiten Konnektor 147' zu dem ersten Konnektor 145' durch die Rückstromsperre verhindert werden.

Da bei einer solchen fehlerhaften Verbindung, etwa einem falschen Stecken der Konnektoren, das heißt einem Vertauschen der Konnektoren beim Verbinden der beiden Leitungen 102, 104 mit der Kurzschlussleitung 100 keine Fluidströmung über die Kurzschlussleitung 100 oder entlang dieser ermöglicht wird, kommt es zu einem Druckanstieg in der Dialysierflüssigkeitszulaufleitung 104, wenn mittels z. B. der ersten Flusspumpe 159 als Fördervorrichtung Fluid entlang der Dialysierflüssigkeitszulaufleitung 104 in Richtung zur Kurzschlussleitung 100 gefördert wird. Dieser Druckanstieg kann beispielsweise durch den Drucksensor S03 erfassten Druck detektiert werden. Beispielsweise kann die Änderung des Drucks zwischen aufeinander folgenden Zeitpunkten, etwa dem ersten und dem zweiten Zeitpunkt ermittelt und als Druckwert verwendet werden. Übersteigt der die Druckänderung anzeigende Druckwert einen ersten Referenzwert, so kann hier auf eine fehlerhafte Verbindung als Zustand geschlossen werden. Ein entsprechendes Signal kann abgegeben werden.

In einer alternativen Ausführungsform wird Fluid mittels der zweiten Flusspumpe 169 und/oder der Ultrafiltrationspumpe 131 gefördert und der Druck in der Dialysatablaufleitung 102 überprüft oder überwacht. Kommt es dabei zu einem - beispielsweise gemessen an einem Referenzwert ausreichend großen - Druckabfall in der Dialysatablaufleitung 102, so kann wiederum auf eine fehlerhafte Verbindung geschlossen werden, da sich die Rückstromsperre bei korrekter Verbindung der Kurzschlussleitung 100 mit den Konnektoren 145 und 147 öffnen sollte, was sie angesichts des Druckabfalls aber erkennbar nicht tut. Ein entsprechendes Signal, das auf eine "fehlerhafte Verbindung" als Zustand hinweist, kann abgegeben werden.

Ebenso kann der Zustand "fehlerhafte Verbindung" durch einen Vergleich in der Dialysierflüssigkeitszulaufleitung 104 herrschenden Drucks mit dem in der Dialysatablaufleitung 102 herrschenden Druck erkannt werden. Übersteigt der Druckunterschied (als Beispiel eines Druckwerts) zwischen dem in der Dialysierflüssigkeitszulaufleitung 104 gemessenen Druck und dem in der Dialysatablaufleitung 102 gemessenen Druck einen vorgegebenen Referenzwert, etwa einen zweiten Referenzwert, so kann ebenfalls auf eine fehlerhafte Verbindung geschlossen werden.

Durch Auswerten von Druckwerten, die auf Drücken, wie sie beispielsweise mittels des Drucksensors S03 oder der Drucksensoren S03 sowie PS4 gemessen wurden, basieren, und durch ihren Vergleich mit einem oder mehreren Referenzwerten, z. B. dem ersten, bzw. zweiten Referenzwert, kann eine fehlerhafte Verbindung als Zustand detektiert und gespeichert und/oder einem Benutzer angezeigt werden.

Beispielsweise kann nach einer detektierten fehlerhaften Verbindung ein Desinfizierungsprogramm in der Blutbehandlungsvorrichtung 2000 durchgeführt werden. Eine Veranlassung hierzu kann von der Steuervorrichtung 150 ausgehen. Diese kann zur automatischen Veranlassung einer solchen Desinfizierung programmiert sein.

Ergänzend oder alternativ kann als Zustand der Kurzschlussleitung 100, beziehungsweise der Rückstromsperre 149, detektiert werden, ob oder dass diese - ggf. trotz korrekter Verbindung, siehe oben - nicht korrekt oder nicht wie beabsichtigt funktioniert, sondern z. B. höhere Drücke zum Öffnen benötigt als vom Hersteller beabsichtigt, was ebenfalls einen Zustand beschreibt. Zur Überprüfung auf einen solchen Zustand kann ein nachfolgend beschriebenes Verfahren durchgeführt werden.

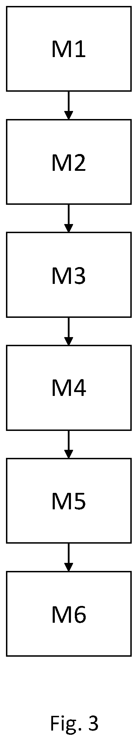

**Fig. 3** zeigt schematisch vereinfacht den Ablauf des erfindungsgemäßen Verfahrens in einer exemplarischen Ausführungsform. Die angeführten Bezugszeichen beziehen sich auf die Beschreibungen zu den Fig. 1 und 2.

Das erfindungsgemäße Verfahren umfasst in dieser Ausführungsform zu einem ersten Zeitpunkt als M1 den Schritt eines Messens oder anderweitigen Ermittelns eines innerhalb der Dialysierflüssigkeitszulaufleitung 104 und/oder innerhalb der Dialysatablaufleitung 102 herrschenden Drucks, hierin auch als Ausgangsdruck bezeichnet.

Dabei kann z. B. festgestellt werden, dass vor dem Schritt M2 am ersten Drucksensor S03 und am zweiten Drucksensor PS4 gleiche oder im Wesentlichen gleiche Drücke anliegen.

Der Schritt M1 ist optional.

M2 repräsentiert den Schritt des Betätigens wenigstens einer Fördervorrichtung, welche hier exemplarisch angeordnet ist zum Fördern von Fluid innerhalb der Dialysatablaufleitung 102, mit dem Ziel, Druck oder Unterdruck gegen die Rückstromsperre der Kurzschlussleitung 100 aufzubauen. Das Betätigen der wenigstens einen Fördervorrichtung erfolgt vorzugsweise bei einem ersten geöffneten Ventil V24, welches hier exemplarisch stromauf des ersten Konnektors 145, aber vorzugsweise stromab des ersten Drucksensors S03 angeordnet ist, und vorzugsweise bei einem geöffneten oder geschlossenen zweiten Ventil V25, welches z. B. stromab des zweiten Konnektors 147, aber vorzugsweise stromauf des zweiten Drucksensors PS4, angeordnet ist.

Die wenigstens eine Fördervorrichtung kann die Ultrafiltrationspumpe 131 und/oder eine zweite Flusspumpe oder Ladepumpe 169 sein.

Das Betätigen der wenigstens einen Fördereinrichtung kann ein Aufbauen eines Unterdrucks oder Drucks sowohl stromauf des ersten Konnektors 145 als auch stromab des zweiten Konnektors 147 umfassen. Hierbei kann ein Unterdruck zwischen 150 hPa und 400 hPa (-150 hPa bis -400 hPa), bevorzugt ein Unterdruck zwischen 250 hPa und 350 hPa (-250 hPa bis -350 hPa, noch bevorzugter ein Unterdruck von 300 hPa (-300 hPa) eingestellt werden. Zu diesem Zeitpunkt, zu welchem dieser Unterdruck aufgebaut wird oder ist, sind die Dialysierflüssigkeitszulaufleitung 104 sowie die Dialysatablaufleitung 102 mit der Kurzschlussleitung 100 verbunden. Durch Betreiben der Fördereinrichtung stromab des Drucksensors PS4 kann somit ein Unterdruck in zumindest einem Teil der Dialysierflüssigkeitszulaufleitung 104, der Kurzschlussleitung 100 und/oder der Dialysatablaufleitung 102 eingestellt werden.

Das Verfahren umfasst als M3 den Schritt eines Messens oder Ermittelns des innerhalb der Dialysierflüssigkeitszulaufleitung 104 und/oder innerhalb der Dialysatablaufleitung 102 herrschenden Drucks, zu einem zweiten Zeitpunkt, welcher zeitlich nach dem ersten Zeitpunkt liegt.

Nach dem zweiten Zeitpunkt kann stromab des zweiten Konnektors 147 und/oder zur Dialysatablaufleitung 102 eine Verbindung zur Atmosphäre hergestellt werden.

Nach dem Entspannen des Unterdrucks in der Dialysatablaufleitung 102 erfolgt ein erneutes Messen des Drucks in der Dialysierflüssigkeitszulaufleitung 104 sowie in der Dialysatablaufleitung 102 zu einem dritten Zeitpunkt.

Das Entspannen des Unterdrucks in der Dialysatablaufleitung 102 kann durch Öffnen der Dialysatablaufleitung 102 zur Umgebung erfolgen. Dabei kann das Entspannen durch Öffnen der Dialysatablaufleitung 102 zum Abfluss 153 über ein Öffnen einer Verbindung mit einer Drain- oder Abflussleitung erfolgen. Alternativ kann das Entspannen des Unterdrucks durch Öffnen der Dialysatablaufleitung 102 zu einem Frischwasserzweig erfolgen. Dabei kann eine Verbindung der Dialysatablaufleitung 102 mit einem Frischwasserzweig nach oder stromab der Wasserquelle 155 und vor oder stromauf der Zuführung der Konzentrate über eine hier nicht dargestellte Verbindungsstelle erfolgen.

Während des Einstellens sowie des Entspannens des Unterdrucks in der Dialysatablaufleitung 102 erfolgt vorzugsweise kein Nachbefüllen der Dialysierflüssigkeitszulaufleitung 104 mit Dialysierflüssigkeit. Dies kann beispielsweise durch Stoppen der Vorrichtung zur Bilanzierung 161 erfolgen.

Das Messen, zum ersten Zeitpunkt und/oder zum zweiten und/oder zum dritten Zeitpunkt, des innerhalb der Dialysierflüssigkeitszulaufleitung 104 und/oder innerhalb der Dialysatablaufleitung 102 herrschenden Drucks kann mittels eines stromauf des ersten Konnektors 145 angeordneten ersten Drucksensors S03 und/oder mittels eines stromab des zweiten Konnektors 147 angeordneten zweiten Drucksensors PS4 erfolgen.

M4 repräsentiert ein Ermitteln einer Veränderung des Drucks innerhalb der Dialysierflüssigkeitszulaufleitung 104 und/oder innerhalb der Dialysatablaufleitung 102 anhand der zum ersten Zeitpunkt und/oder der zum zweiten Zeitpunkt und/oder der zum dritten Zeitpunkt durch Messen des herrschenden Drucks jeweils erhaltenen Messwerte. Die ermittelte Veränderung des Drucks geht als Druckwert in die nachstehende Betrachtung ein.

M5 entspricht dem Schritt eines Auswertens der ermittelten Veränderung mit vorgegebenen Bedingungen. Solche Bedingungen können beispielsweise Grenzwerte, Schwellenwerte, Referenzwerte oder -bereiche, Wertebereiche für die ermittelte Veränderung sein oder umfassen, aber z. B. auch die Dauer, bis eine vorbestimmte Veränderung eintritt, erfasst wird, usw.

Wird zum dritten Zeitpunkt, das heißt nach dem Entspannen des Unterdrucks in der Dialysatablaufleitung 102, ermittelt, dass sich nicht nur der Unterdruck in der Dialysatablaufleitung 102, sondern auch der Unterdruck in der Dialysierflüssigkeitszulaufleitung 104 entspannt hat, so kann auf einen fehlerhaften Zustand oder eine fehlerhafte Funktion der Kurzschlussleitung 100 als Zustand schlussgefolgert werden. Ein fehlerhafter Zustand der Kurzschlussleitung 100 kann dabei ein Zustand sein, welcher durch einen Defekt der Rückstromsperre 149 der an sich korrekt mit Dialysierflüssigkeitszulaufleitung 104 und Dialysatablaufleitung 102 verbundenen Kurzschlussleitung 100 oder durch ein Nicht-Vorhandensein der Rückstromsperre 149 (also z. B. bei Verwenden einer Kurzschlussleitung ohne Rückstromsperre) verursacht wird.

Das Auswerten kann dabei durch Erfassen des Drucks in der Dialysierflüssigkeitszulaufleitung 104 und/oder in der Dialysatablaufleitung 102 nach dem Entspannen des Unterdrucks erfolgen. Ist die Rückstromsperre 149 defekt oder nicht vorhanden, so kann ein Rückstrom von der Dialysatablaufleitung 102 in Richtung der Dialysierflüssigkeitszulaufleitung 104 erfolgen. Folglich erhöht sich nicht nur der Druck in der Dialysatablaufleitung 102 sondern auch, und ggf. in selbem Maße oder Umfang, in der Dialysierflüssigkeitszulaufleitung 104.

Wie oben beschrieben kann eine Druckerfassung zu drei Zeitpunkten erfolgen. Alternativ kann die Auswertung auch auf einem Vergleich des Drucks als Druckwert in der Dialysierflüssigkeitszulaufleitung 104 zu dem zweiten Zeitpunkt, das heißt nach dem Aufbau des Unterdrucks in der Dialysatablaufleitung 102, und zum dritten Zeitpunkt, das heißt nach dem Entspannen des Unterdrucks in der Dialysatablaufleitung 102, erfolgen. Zeigt ein Vergleich der zu diesen Zeitpunkten in der Dialysierflüssigkeitszulaufleitung 104 vorliegenden Drücke, z. B. miteinander oder mit einem vorausgehenden Druck, beispielsweise ein Überschreiten eines Grenzwertes, das heißt z. B. ein Erhöhen des Drucks in der Dialysierflüssigkeitszulaufleitung 104, so kann auf ein Rückströmen von Fluid aus der Dialysatablaufleitung 102 in die Dialysierflüssigkeitszulaufleitung 104 geschlossen werden. Ein fehlerhafter Zustand der Kurzschlussleitung 100 liegt somit vor.

Damit ist erfindungsgemäß und insbesondere bei dieser alternativen Ausführungsform ein Erfassen des Drucks nur in der Dialysierflüssigkeitszulaufleitung 104 und/oder vorzugsweiseweise nur zu einem Zeitpunkt nach dem Einstellen eines Unterdrucks in der Dialysatablaufleitung 102 sowie zu einem Zeitpunkt nach dem Entspannen des Unterdrucks in der Dialysatablaufleitung 102 ausreichend.

In einer weiteren alternativen Ausführungsform kann die Auswertung basierend auf einem Vergleich des Drucks in der Dialysierflüssigkeitszulaufleitung 104 nach dem Entspannen des Unterdrucks (nach dem dritten Zeitpunkt) mit einem Druck in der Dialysatablaufleitung 102 erfolgen. Liegt die Druckdifferenz dieser Drücke als Druckwert z. B. unterhalb eines Grenzwertes, so kann auf einen fehlerhaften Zustand der Kurzschlussleitung 100 geschlussfolgert werden. Da bei einem fehlerfreien Zustand der Rückstromsperre 149 kein Rückstrom stattfindet, bleibt der Unterdruck in der Dialysierflüssigkeitszulaufleitung 104 erhalten, während sich der Unterdruck in der Dialysatablaufleitung 102 entspannt, wodurch eine Druckdifferenz zwischen der Dialysierflüssigkeitszulaufleitung 104 und Dialysatablaufleitung 102 eingestellt wird oder sich einstellt.

In einer weiteren alternativen Ausführungsform wird nach dem dritten Zeitpunkt, das heißt dem Entspannen des Unterdrucks in der Dialysatablaufleitung 102, diese stromab abgesperrt, so dass keine fluidische Verbindung zum Abfluss 153 oder zur Frischwasserquelle 155 besteht. Anschließend wird die Druckveränderung als Druckwert in der Dialysatablaufleitung 102, durch zumindest zwei Druckmessungen ermittelt. Zeigt sich ein Absinken des Drucks in der Dialysatablaufleitung 102, so kann ebenso auf einen fehlerhaften Zustand der Kurzschlussleitung 100 geschlussfolgert werden, da ein Rückströmen über die Rückstromsperre 149 in die Dialysierflüssigkeitszulaufleitung 104, in welcher noch Unterdruck herrscht, erfolgt. Ein entsprechendes Signal kann jeweils abgegeben werden.

M6 repräsentiert schließlich den optionalen Schritt des Ausgebens eines entsprechenden Signals, beispielsweise einer Mitteilung oder eines Alarms, an den Benutzer der Behandlungsvorrichtung 2000, ob die Veränderung den vorbestimmten Bedingungen genügt oder eben nicht, das heißt ob ein fehlerhafter Zustand der Kurzschlussleitung 100 ermittelt wurde oder eben nicht. Dieses Signal kann in manchen Ausführungsformen auch Hinweise an den Benutzer umfassen, beispielsweise, dass die Hydraulik vor der nächsten Benutzung einer Desinfektion bedarf.

Dieses Signal kann in manchen Ausführungsformen der Steuervorrichtung zugeführt werden und über diese, ausgelöst von dieser, zu einer Desinfektion, zu einem Sperren der Benutzung der Behandlungsvorrichtung oder mancher Betriebsmodi vor einer nächsten Behandlung, vor einer nächsten Reinigung oder Desinfizierung oder dergleichen führen, usw.

### Bezugszeichenliste

- 100: Kurzschlussleitung

- 2000: medizintechnische Behandlungsvorrichtung
- 2001: Gehäuse

- 101: Blutpumpe
- 102: Dialysatablaufleitung
- 104: Dialysierflüssigkeitszulaufleitung
- 105: Substituatleitung
- 107: Prädilutionsventil
- 107a: zum Prädilutionsventil gehörige Leitung
- 109: Postdilutionsventil
- 109a: zum Postdilutionsventil gehörige Leitung
- 111: Substituatpumpe
- 131: Ultrafiltrationspumpe
- 145: Konnektor der Dialysierflüssigkeitszulaufleitung
- 145': erster Konnektor der Kurzschlussleitung
- 147: Konnektor der Dialysatablaufleitung
- 147': zweiter Konnektor der Kurzschlussleitung
- 149: Rückstromsperre; Rückschlagventil

- 150: Steuervorrichtung
- 153: Abfluss
- 155: Wasserquelle
- 157: Wärmetauscher
- 159: erste Flusspumpe
- 161: Vorrichtung zur Bilanzierung
- 162: Heizvorrichtung
- 163: Anmischvorrichtung
- 163a: Leitfähigkeitssensor
- 163b: Leitfähigkeitssensor
- 165a: Temperatursensor
- 165b: Temperatursensor
- 166: Konzentratversorgung
- 168: Konzentratversorgung
- 169: zweite Flusspumpe
- 171: Pumpe, Natriumpumpe
- 173: Pumpe, Bicarbonatpumpe

- 300: extrakorporaler Blutkreislauf
- 301: erste Leitung (arterieller Leitungsabschnitt)
- 302: (erste) Schlauchklemme
- 303: Blutfilter oder Dialysator
- 303a: Dialysierflüssigkeitskammer
- 303b: Blutkammer
- 303c: semi-permeable Membran
- 305: zweite Leitung (venöser Leitungsabschnitt)
- 306: (zweite) Schlauchklemme
- 315: Detektor
- 317: Single-Needle-Kammer
- 318: Entlüftungseinrichtung
- 319: Detektor
- 325: Zugabestelle für Heparin
- 329: venöse Blutkammer (optional)

- F1: Filter
- F2: Filter
- A: Behälter
- B: Behälter

- Ä: Äußeres
- I: Inneres
- M1 bis M6: Verfahrensschritte

- P: Druckmessstellen
- PS1: arterieller Drucksensor (optional)
- PS2: arterieller Drucksensor (optional)
- PS3: Drucksensor (optional)
- PS4: zweiter Drucksensor zum Messen des Filtratdrucks (optional)

- S03: erster Drucksensor

- V: Ventile

- V24: Ventil in Dialysierflüssigkeitszulaufleitung
- V25: Ventil in Dialysatablaufleitung

- VB: Bypassventile
- Y: Y-Verbinder

## Patentansprüche

1. Verfahren zum Überprüfen eines Zustands einer mit einer Dialysierflüssigkeitszulaufleitung (104) und einer Dialysatablaufleitung (102) einer bereitgestellten medizintechnischen Behandlungsvorrichtung (2000), welche
- die Dialysierflüssigkeitszulaufleitung (104) mit einem zu ihrer Verbindung mit einem Blutfilter (303) vorgesehenen Konnektor (145);
- die Dialysatablaufleitung (102) mit einem zu ihrer Verbindung mit einem Blutfilter (303) vorgesehenen Konnektor (147); und
- eine Fördervorrichtung (159, 169, 131) zum Fördern von Fluid innerhalb der Dialysierflüssigkeitszulaufleitung (104) und/oder innerhalb der Dialysatablaufleitung (102);
aufweist, verbundenen Kurzschlussleitung (100), welche wenigstens aufweist:
- einen ersten Konnektor (145') zum Verbinden der Kurzschlussleitung (100) unter Erzielen einer Fluidverbindung mit dem Konnektor (145) der Dialysierflüssigkeitszulaufleitung (104);
- einen zweiten Konnektor (147') zum Verbinden der Kurzschlussleitung (100) unter Erzielen einer Fluidverbindung mit dem Konnektor (147) der Dialysatablaufleitung (102);
- eine Rückstromsperre (149), welche das Durchströmen der Kurzschlussleitung (100) nur vom ersten Konnektor (145') in Richtung zum zweiten Konnektor (147') zulässt,
wobei das Verfahren umfasst:
- Betätigen der Fördervorrichtung (159, 169, 131) mit dem Ziel, Fluid innerhalb der Dialysierflüssigkeitszulaufleitung (104) und/oder innerhalb der Dialysatablaufleitung (102) zu fördern zum Aufbauen von Überdruck oder Unterdruck darin;
- Messen eines in der Dialysierflüssigkeitszulaufleitung (104) und/oder in der Dialysatablaufleitung (102) herrschenden Drucks zu einem ersten Zeitpunkt;
- Feststellen wenigstens eines Druckwerts basierend auf dem gemessenen Druck; und
- Auswerten des wenigstens einen Druckwerts, und
- Abgeben oder Ausgeben eines Signals, den Zustand der Kurzschlussleitung (100) angebend, als Ergebnis des Auswertens, wobei
- das Ergebnis eine fehlerhafte Verbindung der Kurzschlussleitung (100) mit der Dialysierflüssigkeitszulaufleitung (104) und/oder der Dialysatablaufleitung (102) ist, und/oder
- das Ergebnis eine fehlerhafte Funktion der Rückstromsperre (149) der Kurzschlussleitung (100) ist.

2. Verfahren nach Anspruch 1,
- wobei das Auswerten des wenigstens einen Druckwerts ein Vergleichen des wenigstens einen Druckwerts mit einem ersten Referenzwert oder -bereich ist oder umfasst.

3. Verfahren nach Anspruch 1 oder **2,** wobei
- das Ergebnis, dass eine fehlerhafte Verbindung vorliegt, eine Verbindung des Konnektors (145) der Dialysierflüssigkeitszulaufleitung (104) mit dem zweiten Konnektor (147') der Kurzschlussleitung (100) und eine Verbindung des Konnektors (147) der Dialysatablaufleitung (102) mit dem ersten Konnektor (145') der Kurzschlussleitung (100) ist oder voraussetzt.

4. Verfahren nach einem der Ansprüche 1 bis **3,**
wobei zum Feststellen des Druckwerts sowohl der zum ersten Zeitpunkt in der Dialysierflüssigkeitszulaufleitung (104) herrschende Druck als auch der in der Dialysatablaufleitung (102) herrschende Druck gemessen wird oder wurde, wobei zwischen dem in der Dialysierflüssigkeitszulaufleitung (104) gemessenen Druck und dem in der Dialysatablaufleitung (102) gemessenen Druck, oder umgekehrt, eine Druckdifferenz ermittelt wird;
wobei das Auswerten des wenigstens einen Druckwerts ein Vergleichen dieser Druckdifferenz mit einem zweiten Referenzwert oder -bereich hierfür ist oder umfasst.

5. Verfahren nach einem der Ansprüche 1 bis 4, wobei das Verfahren als weiteren Schritt umfasst:
- Messen eines in der Dialysierflüssigkeitszulaufleitung (104) und/oder in der Dialysatablaufleitung (102) herrschenden Drucks zu einem zweiten Zeitpunkt und Feststellen wenigstens eines zweiten Druckwerts hierauf basierend;
wobei das Auswerten des Druckwerts ein Vergleichen der Druckdifferenz zwischen den in der Dialysierflüssigkeitszulaufleitung (102) zum ersten und zweiten Zeitpunkt festgestellten Druckwerten und/oder ein Vergleichen der Druckdifferenz zwischen den in der Dialysatablaufleitung (104) zum ersten und zweiten Zeitpunkt festgestellten Druckwerten mit jeweils einem dritten bzw. vierten Referenzwert oder -bereich hierfür ist oder umfasst.

6. Verfahren nach einem der Ansprüche 1 bis 5,
wobei das Betätigen der Fördereinrichtung mit dem Ziel des Aufbauens eines Unterdrucks in der Dialysierflüssigkeitszulaufleitung (104) sowie in der Dialysatablaufleitung (102) erfolgt; und
wobei das Verfahren den weiteren Schritt umfasst:
- Entspannen des aufgebauten Unterdrucks in der Dialysatablaufleitung (102);
wobei das Messen eines in der Dialysierflüssigkeitszulaufleitung (104) und/oder in der Dialysatablaufleitung (102) herrschenden Drucks ein Messen zu einem oder mehr Zeitpunkten nach dem Entspannen umfasst oder hieraus besteht.

7. Verfahren nach einem der Ansprüche 1 bis 6,
wobei das Betätigen der wenigstens einen Fördervorrichtung bei einem geöffneten ersten Ventil (V24), welches stromauf des Konnektors (145) der Dialysierflüssigkeitszulaufleitung (104), aber stromab eines ersten Drucksensors (S03), und/oder bei einem geöffneten zweiten Ventil (V25), welches stromab des zweiten Konnektors (147) der Dialysatablaufleitung (102), aber stromauf eines zweiten Drucksensors (PS4), angeordnet ist, erfolgt.

8. Verfahren nach einem der Ansprüche 6 bis 7, wobei
das Entspannen des Unterdrucks in der Dialysatablaufleitung (102) vorzugsweise durch ein Verbinden der Dialysatablaufleitung (102) mit einer Frischwasserquelle (155) oder einem Abfluss (153) erfolgt, noch bevorzugter mittels Öffnen eines Ventils zu einer Frischwasserleitung oder eines Ventils zu einer Drain- oder Abflussleitung erfolgt oder dieses umfasst.

9. Verfahren nach einem der Ansprüche 6 bis 8,
wobei ein Signal ab- oder ausgegeben wird, welches einem fehlerhaften Zustand der Kurzschlussleitung (100) zugeordnet ist, wenn
- das Auswerten des Druckwerts dessen Überschreiten eines Referenzwertes oder Verlassen eines Referenzbereichs ergibt, und/oder
- wenn das Feststellen des Druckwerts basierend auf nach der Entspannung innerhalb der Dialysierflüssigkeitszulaufleitung (104) gemessenen Drücken eine Druckerhöhung ergibt.

10. Verfahren nach einem der Ansprüche 6 bis 9, weiter umfassend:
- fluidisches Absperren der Dialysatablaufleitung (102) nach dem Entspannen des Unterdrucks in der Dialysatablaufleitung (102) derart, dass keine Verbindung der Dialysatablaufleitung (102) zur Frischwasserquelle (155) oder dem Abfluss (153) mehr besteht,
wobei zum Feststellen des wenigstens einen Druckwerts zumindest zwei Drücke in der Dialysatablaufleitung (102) zu zwei unterschiedlichen Zeitpunkten nach ihrem Sperren gemessen werden, und
wobei ein Signal ab- oder ausgegeben wird, welches einem fehlerhaften Zustand der Kurzschlussleitung (100) zugeordnet ist, wenn als Ergebnis des Auswertens des wenigstens einen Druckwerts eine Druckänderung innerhalb der Dialysatablaufleitung (102) festgestellt wird.

11. Steuervorrichtung (150), programmiert zum Ausführen eines Tests zum Überprüfen eines Zustands einer Kurzschlussleitung (100), verbunden mit der Dialysierflüssigkeitszulaufleitung (104) und der Dialysatablaufleitung (102) einer medizintechnischen Blutbehandlungsvorrichtung (2000), wobei der Test die Schritte eines der Verfahren gemäß einem der Ansprüche 1 bis 10 umfasst.

12. Medizintechnische Behandlungsvorrichtung (2000),
- aufweisend eine Steuervorrichtung (150) nach Anspruch 11.

## Claims

1. A method for checking a condition of a bypass line (100) connected to a dialysis liquid inlet line (104) and a dialysate outlet line (102) of a provided medical-technical treatment apparatus (2000), wherein the medical-technical treatment apparatus (2000) comprises:
- the dialysis liquid inlet line (104) with a connector (145) provided for its connection to a blood filter (303);
- the dialysate outlet line (102) with a connector (147) provided for its connection to a blood filter (303); and
- a conveying device (159, 169, 131) for conveying fluid within the dialysis liquid inlet line (104) and/or within the dialysate outlet line (102);
wherein the connected bypass line (100) comprises at least:
- a first connector (145') for connecting the bypass line (100) to achieve fluid connection with the connector (145) of the dialysis liquid inlet line (104);
- a second connector (147') for connecting the bypass line (100) to achieve fluid connection with the connector (147) of the dialysate outlet line (102);
- a non-return valve (149) which allows the flow through the bypass line (100) only from the first connector (145') towards the second connector (147'),
wherein the method comprises:
- actuating the conveying device (159, 169, 131) with the aim of conveying fluid within the dialysis liquid inlet line (104) and/or within the dialysate outlet line (102) to establish positive pressure or negative pressure therein;
- measuring a pressure prevailing in the dialysis liquid inlet line (104) and/or in the dialysate outlet line (102) at a first time point;
- determining at least one pressure value based on the measured pressure; and
- evaluating the at least one pressure value; and
- emitting or outputting a signal indicating the condition of the bypass line (100) as a result of the evaluation, wherein:
- the result is a faulty connection of the bypass line (100) with the dialysis liquid inlet line (104) and/or the dialysate outlet line (102), and/or
- the result is a faulty function of the non-return valve (149) of the bypass line (100).

2. The method according to claim 1,
- wherein evaluating of the at least one pressure value is or comprises a comparison of the at least one pressure value with a first reference value or reference range.

3. The method according to claim 1 or 2, wherein
- the result that a faulty connection exists, is or presupposes a connection of the connector (145) of the dialysis liquid inlet line (104) to the second connector (147') of the bypass line (100) and a connection of the connector (147) of the dialysate outlet line (102) to the first connector (145') of the bypass line (100).

4. The method according to any one of claims 1 to 3,
wherein, in order to determine the pressure value, both the pressure prevailing in the dialysis liquid inlet line (104) at the first time point, and the pressure prevailing in the dialysate outlet line (102) are or have been measured, wherein a pressure difference is determined between the pressure measured in the dialysis liquid inlet line (104) and the pressure measured in the dialysate outlet line (102), or vice versa;
wherein evaluating the at least one pressure value is or comprises a comparison of this pressure difference with a second reference value or reference range therefor.

5. The method according to any one of claims 1 to 4, wherein the method further comprises:
- measuring a pressure prevailing in the dialysis liquid inlet line (104) and/or in the dialysate outlet line (102) at a second time point and determining at least a second pressure value based thereon;
wherein evaluating the pressure value is or comprises a comparison of the pressure difference between the pressure values determined in the dialysis liquid inlet line (104) at the first and second time points and/or a comparison of the pressure difference between the pressure values determined in the dialysate outlet line (102) at the first and second time points, with respectively a third or fourth reference value or range therefor.

6. The method according to any one of claims 1 to 5,
wherein actuating the conveying device is carried out with the aim of establishing a negative pressure in the dialysis liquid inlet line (104) and in the dialysate outlet line (102);
and wherein the method further comprises:
- releasing the established negative pressure in the dialysate outlet line (102);
wherein measuring a pressure prevailing in the dialysis liquid inlet line (104) and/or in the dialysate outlet line (102) comprises or consists of measuring at one or more time points after the pressure release.

7. The method according to any one of claims 1 to 6,
wherein actuating the at least one conveying device takes place when a first valve (V24) is open, which is arranged upstream of the connector (145) of the dialysis liquid inlet line (104) but downstream of a first pressure sensor (S03), and/or when a second valve (V25) is open, which is arranged downstream of the second connector (147) of the dialysate outlet line (102) but upstream of a second pressure sensor (PS4).

8. The method according to any one of claims 6 to 7,
wherein releasing the negative pressure in the dialysate outlet line (102) is preferably achieved by connecting the dialysate outlet line (102) to a fresh water source (155) or to a drain (153), more preferably by opening a valve to a fresh water line or a valve to a drain or discharge line, or comprises this.

9. The method according to any one of claims 6 to 8,
wherein a signal, associated with a faulty condition of the bypass line (100), is emitted or output
- if the evaluation of the pressure value indicates that it exceeds a reference value or leaves a reference range, and/or
- if the determination of the pressure value, based on pressures measured after release within the dialysis liquid inlet line (104), indicates a pressure increase.

10. The method according to any one of claims 6 to 9, further comprising
- fluidically shutting off the dialysate outlet line (102) after the negative pressure in the dialysate outlet line (102) has been released in such a way that there is no longer any connection between the dialysate outlet line (102) and the fresh water source (155) or the drain (153),
wherein in order to determine the at least one pressure value, at least two pressures in the dialysate outlet line (102) are measured at two different time points after it has been shut off, and
wherein a signal, associated with a faulty condition of the bypass line (100), is emitted or output, when, as a result of evaluating the at least one pressure value,
a pressure change is detected within the dialysate outlet line (102).

11. A control device (150), programmed to carry out a test to check a condition of a bypass line (100) connected to the dialysis liquid inlet line (104) and the dialysate outlet line (102) of a medical-technical blood treatment apparatus (2000), the test comprising the steps of any one of the methods according to any one of claims 1 to 10.

12. A medical technical treatment apparatus (2000),
- comprising a control device (150) according to claim 11.

## Revendications

1. Un procédé de vérification d'un état d'une conduite de dérivation (100) raccordée à une conduite d'alimentation en liquide de dialyse (104) ainsi qu'à une conduite d'évacuation de dialysat (102) d'un appareil médico-technique de traitement (2000) fourni, où l'appareil médico-technique de traitement (2000) comprend :
- la conduite d' alimentation en liquide de dialyse (104) munie d'un connecteur (145) prévu pour son raccordement à un filtre sanguin (303);
- la conduite d'évacuation du dialysat (102) munie d'un connecteur (147) prévu pour son raccordement à un filtre sanguin (303); et
- un dispositif d'acheminement (159, 169, 131) pour l'acheminement de fluide à l'intérieur de la conduite d'alimentation en liquide de dialyse (104) et/ou à l'intérieur de la conduite d'évacuation du dialysat (102);
où la conduite de dérivation (100) raccordée, comprend au moins :
- un premier connecteur (145') permettant de raccorder la conduite de dérivation (100) afin d'établir une communication fluidique avec le connecteur (145) de la conduite d'alimentation en liquide de dialyse (104);
- un second connecteur (147') permettant de raccorder la conduite de dérivation (100) afin d'établir une communication fluidique avec le connecteur (147) de la conduite d'évacuation du dialysat (102);
- un clapet anti-retour (149) qui permet l'écoulement à travers la conduite de dérivation (100) uniquement du premier connecteur (145') vers le second connecteur (147') ,
où le procédé comprend les étapes suivantes :
- actionner le dispositif d'acheminement (159, 169, 131) dans le but d'acheminer du fluide dans la conduite d'alimentation en liquide de dialyse (104) et/ou dans la conduite d'évacuation du dialysat (102) pour y créer une pression positive ou une pression négative;
- mesurer la pression régnant dans la conduite d'alimentation en liquide de dialyse (104) et/ou dans la conduite d'évacuation du dialysat (102) à un premier instant.
- déterminer au moins une valeur de pression sur la base de la pression mesurée; et
- évaluer la au moins une valeur de pression; et
- émettre ou délivrer un signal indiquant l'état de la conduite de dérivation (100) à la suite de l'évaluation, où :
- le résultat est un raccordement défectueux de la conduite de dérivation (100) avec la conduite d'alimentation en liquide de dialyse (104) et/ou la conduite d'évacuation du dialysat (102), et/ou
- le résultat est un dysfonctionnement du clapet anti-retour (149) de la conduite de dérivation (100).

2. Le procédé selon la première revendication,
- où l'évaluation d'au moins une valeur de pression consiste en ou comprend une comparaison d'au moins une valeur de pression avec une première valeur de référence ou plage de référence.

3. Le procédé selon la revendication 1 ou 2, où
- le résultat, qu'un raccordement défectueux existe, consiste en ou présuppose un raccordement du connecteur (145) de la conduite d'alimentation en liquide de dialyse (104) avec le second connecteur (147') de la conduite de dérivation (100) ainsi qu'un raccordement du connecteur (147) de la conduite d'évacuation du dialysat (102) avec le premier connecteur (145') de la conduite de dérivation (100).

4. Le procédé selon l'une quelconque des revendications 1 à 3,
- où, pour déterminer la valeur de pression, on mesure ou on a mesuré tant la pression régnant dans la conduite d'alimentation en liquide de dialyse (104) au premier instant, que la pression régnant dans la conduite d'évacuation du dialysat (102), où une différence de pression est déterminée entre la pression mesurée dans la conduite d'alimentation en liquide de dialyse (104) et la pression mesurée dans la conduite d'évacuation du dialysat (102), ou inversement;
- où l'évaluation d'au moins une valeur de pression consiste en ou comprend une comparaison de cette différence de pression avec une seconde valeur de référence ou plage de référence pour celle-ci.

5. Le procédé selon l'une quelconque des revendications 1 à 4, où le procédé comprend les étapes suivantes :
- mesurer une pression régnant dans la conduite d'alimentation en liquide de dialyse (104) et/ou la conduite d'évacuation du dialysat (102) à un second instant et déterminer au moins une seconde valeur de pression sur cette base;
où l'évaluation de la valeur de pression est ou comprend une comparaison de la différence de pression entre les valeurs de pression déterminées dans la conduite d'alimentation en liquide de dialyse (104) aux premier et second instants et/ou une comparaison de la différence de pression entre les valeurs de pression déterminées dans la conduite d'évacuation du dialysat (102) aux premier et seconds instants avec respectivement une troisième ou une quatrième valeur de référence ou plage de référence à cet effet.

6. Le procédé selon l'une quelconque des revendications 1 à 5,
où l'actionnement du dispositif d'acheminement est effectué dans le but d'établir une pression négative dans la conduite d'alimentation en liquide de dialyse (104) ainsi que dans la conduite d'évacuation du dialysat (102); et
où le procédé comprend en outre l'étape suivante :
- relâcher la pression négative établie dans la conduite d'évacuation du dialysat (102);
où la mesure d'une pression régnant dans la conduite d'alimentation en liquide de dialyse (104) et/ou dans la conduite d'évacuation du dialysat (102) comprend ou consiste en une mesure à un ou plusieurs instants après le relâchement.

7. Le procédé selon l'une quelconque des revendications 1 à 6,
où l'actionnement d'au moins un dispositif d'acheminement a lieu lorsqu'une première vanne (V24) est ouverte,
laquelle est agencée en amont du connecteur (145) de la conduite d'alimentation en liquide de dialyse (104), mais en aval d'un premier capteur de pression (S03) , et/ou lorsqu'une seconde vanne (V25) est ouverte, laquelle est agencée en aval du second connecteur (147) de la conduite d'évacuation du dialysat (102), mais en amont d'un second capteur de pression (PS4).

8. Le procédé selon l'une quelconque des revendications 6 à 7,
où le relâchement de la pression négative dans la conduite d'évacuation du dialysat (102) est réalisé de préférence en reliant la conduite d'évacuation du dialysat (102) à une source d'eau potable (155) ou à une évacuation (153), encore plus préférablement, en ouvrant une vanne vers une conduite d'eau potable ou une vanne vers une conduite de drainage ou d'évacuation, ou comprend ceci.

9. Le procédé selon l'une quelconque des revendications 6 à 8,
où un signal, associé à un état défectueux de la conduite de dérivation (100), est émis ou délivré
- si l'évaluation de la valeur de pression indique qu'elle dépasse une valeur de référence ou qu'elle sort d'une plage de référence, et/ou
- si la détermination de la valeur de pression, basée sur les pressions mesurées après le relâchement à l'intérieur de la conduite d'alimentation en liquide de dialyse (104), indique une augmentation de la pression.

10. Le procédé selon l'une quelconque des revendications 6 à 9, comprenant en outre :
une fermeture fluidique de la conduite d'évacuation du dialysat (102) après le relâchement de la pression négative dans la conduite d'évacuation du dialysat (102), de telle sorte qu'il n'y ait plus aucune liaison entre la conduite d'évacuation du dialysat (102) et la source d'eau potable (155) ou l'évacuation (153),
où, afin de déterminer au moins une valeur de pression, au moins deux pressions dans la conduite d'évacuation du dialysat (102) sont mesurées à deux instants différents après sa fermeture, et
où un signal, associé à un état défectueux de la conduite de dérivation (100), est émis ou délivré lorsque, à la suite de l'évaluation de la au moins une valeur de pression, un changement de pression est détecté à l'intérieur de la conduite d'évacuation du dialysat (102).

11. Un dispositif de commande (150), programmé pour exécuter un test visant à vérifier l'état d'une conduite de dérivation (100) reliée à la conduite d'alimentation en liquide de dialyse (104) et à la conduite d'évacuation du dialysat (102) d'un appareil médico-technique de traitement du sang (2000), le test comprenant les étapes de l'un quelconque des procédés selon l'une quelconque des revendications 1 à 10.

12. Un appareil médico-technique de traitement du sang (2000),
- comprenant un dispositif de commande (150) selon la revendication 11.
